# EUROPÄISCHE PATENTSCHRIFT

(11) **EP 3 515 991 B1**
(45) Veröffentlichungstag und Bekanntmachung des Hinweises auf die Patenterteilung: **07.10.2020**
(21) Anmeldenummer: 17731064.6
(22) Anmeldetag: 23.05.2017
(51) Int. Cl.: C09B 57/00, C07D 311/92, G02B 5/23, C07D 493/04, C07D 493/14, C07D 495/04

(54) **PHOTOCHROME MEHRFACH-ANNELLIERTE NAPHTHOPYRANE MIT SEHR LANGWELLIGER ABSORPTION BIS WEIT IN DEN SICHTBAREN WELLENBEREICH**
PHOTOCHROMIC MULTIPLE RING-FUSED NAPHTHOPYRANS HAVING EXTREME LONGWAVE ABSORPTION EXTENDING FAR INTO THE VISIBLE WAVELENGTH RANGE
NAPHTHOPYRANNES PHOTOCHROMIQUES À MULTIPLES CONDENSATIONS DE NOYAUX OFFRANT UNE ABSORPTION DE TRÈS GRANDES LONGUEURS D'ONDES ALLANT LOIN DANS LES LONGUEURS D'ONDES DU DOMAINE VISIBLE

(30) Priorität: 26.09.2016 DE 102016011559
(43) Veröffentlichungstag der Anmeldung: 31.07.2019
(73) Patentinhaber: Rodenstock GmbH, 80687 München (DE)
(72) Erfinder: WEIGAND, Udo, 81247 München (DE); ZINNER, Herbert, 85296 Rohrbach (DE)
(74) Vertreter: Müller-Boré & Partner Patentanwälte PartG mbB
(86) Internationale Anmeldenummer: PCT/EP2017/000617
(87) Internationale Veröffentlichungsnummer: WO 2018/054507

(56) Entgegenhaltungen:
- EP-A1- 0 912 908
- EP-A1- 2 178 883
- EP-A1- 2 655 354
- EP-A1- 2 684 886
- EP-A1- 2 760 869
- EP-A1- 2 788 340
- EP-A1- 2 829 537

## Beschreibung

Die vorliegende Erfindung betrifft photochrome mehrfach-annellierte Naphthopyrane mit sehr langwelliger Absorption der geschlossenen Form bis weit in den sichtbaren Wellenlängenbereich hinein sowie deren Verwendung in Kunststoffen aller Art, insbesondere für ophthalmische Zwecke, vorzugsweise in Autofahrer-Brillen. Die erfindungsgemäßen Verbindungen weisen zudem eine sehr gute Lebensdauer bei sehr hoher Leistung auf.

Seit langem sind verschiedene Farbstoffklassen bekannt, die bei Bestrahlung mit Licht bestimmter Wellenlängen, insbesondere Sonnenstrahlen, reversibel ihre Farbe wechseln. Dies geht darauf zurück, dass diese Farbstoffmoleküle durch Lichtenergie in einen angeregten Zustand ("offene Form") übergehen, den sie bei Unterbrechung der Energiezufuhr wieder verlassen und in ihren Ausgangszustand zurückkehren. Zu diesen photochromen Farbstoffen gehören verschiedene Pyransysteme, die im Stand der Technik mit unterschiedlichen Grundsystemen und Substituenten bereits beschrieben wurden.

Pyrane, speziell Naphthopyrane und von diesen abgeleitete größere Ringsysteme, sind derzeit die am meisten bearbeitete Klasse photochromer Verbindungen. Obwohl bereits im Jahr 1966 erstmals zum Patent angemeldet (US 3,567,605), konnten erst in den 90er Jahren Verbindungen entwickelt werden, die für den Einsatz in Brillengläsern geeignet erschienen. Eine geeignete Verbindungsklasse von Pyranen sind zum Beispiel die 2,2-Diaryl-2H-naphtho[1,2-b]pyrane oder die 3,3-Diaryl-3H-naphtho[2,1-b]pyrane, die in angeregter Form verschiedene Eindunklungsfarben von gelb bis rot aufweisen.

Überaus interessant sind von diesen Grundsystemen abgeleitete, annellierte Naphthopyrane, die aufgrund ihres größeren Ringsystems längerwellig absorbieren und rote, violette und blaue Eindunklungsfarben ergeben. Dazu wird üblicherweise ein Benzolring mit einer zusätzlichen Verbrückung in ortho-Stellung (in den nachstehenden erfindungsgemäßen Verbindungen die Brücke mit den Substituenten R₁ und R₂) eingesetzt.

Liegt - wie in den nachstehenden Verbindungen der vorliegenden Erfindung - eine einatomige Verbrückung vor, so ergibt sich ein an das Naphthopyran annellierter Fünfring. Beispiele finden sich in EP 0 792 468 und EP 0 906 366 für ein Kohlenstoff-Brückenatom ("Indenonaphthopyrane") sowie in EP 0 946 536 für ein Sauerstoff-Brückenatom.

In EP 2 457 915, EP 2 471 794, EP 2 684 886, EP 2 788 340 sowie EP 2 872 517 sind Verbindungen offenbart, in denen an die Indenonaphthopyran-Kernstruktur mindestens ein weiteres Ringsystem annelliert ist, jedoch im Gegensatz zu den erfindungsgemäßen Verbindungen der nachstehenden Formel (I) an die C-C-Bindung zwischen den Substituenten R₇ und R₁₀, d.h. an einer anderen Stelle der Kernstruktur. Die Annellierung an dieser Stelle ergibt jedoch eine deutlich geringere bathochrome Verschiebung der längstwelligen Absorptionsbande im Vergleich zu den nachstehenden erfindungsgemäßen Verbindungen der Formel (I).

In EP 0 912 908 sind unter anderem auch Verbindungen aufgeführt, bei denen an die Indenonaphthopyran-Kernstruktur zwei weitere Ringsysteme annelliert sind, d.h. an derselben Stelle wie bei den nachstehenden erfindungsgemäßen Verbindungen der Formel (I). Jedoch bewirken die beschriebenen Annellierungen (Benzofuro, Benzothieno und Indolo) im Vergleich zu den erfindungsgemäßen Verbindungen der Formel (I) eine deutlich geringere bathochrome Verschiebung der längstwelligen Absorptionsbande der geschlossenen Form.

Liegt dagegen eine zweiatomige Verbrückung vor, so ergibt sich ein an das Naphthopyran annellierter Sechsring. Verbindungen mit einer Brücke aus zwei Kohlenstoff-Atomen sind in EP 1 119 560 beschrieben; vgl. auch EP 2 829 537 bzw. EP 3 010 924.

EP 1 097 156 beschreibt Verbindungen mit einer zweiatomigen Brücke aus einem Kohlenstoff- und einem Sauerstoff-Atom, ebenso wie EP 2 178 883 und EP 2 760 869.

Die verschiedenen, im Stand der Technik bislang verfügbaren photochromen Farbstoffe zeigen jedoch keine langwellige Absorption der geschlossenen Form, die bis weit in den violetten bzw. blauen sichtbaren Wellenlängenbereich hineinreicht. Letzteres würde es nämlich ermöglichen, dass mit solchen photochromen Farbstoffen gestaltete Brillengläsern kein oder kaum UV-Licht mehr für den Übergang in die angeregte (farbige) Form benötigen und insofern daher auch ein Eindunkeln solcher Brillengläser z.B. hinter einer komplett UV-absorbierenden Auto-Windschutzscheibe erreicht werden könnte.

Der vorliegenden Erfindung liegt daher die Aufgabe zugrunde, photochrome Farbstoffe bereitzustellen, die sich durch eine langwellige Absorption der geschlossenen Form, die bis weit in den violetten bzw. blauen sichtbaren Wellenlängenbereich hineinreicht, auszeichnen sollen.

Diese Aufgabe wird durch die in den Ansprüchen gekennzeichneten Gegenstände gelöst.

Insbesondere werden photochrome mehrfach-annellierte Naphthopyrane gemäß den allgemeinen Formeln (I), (II) bzw. (III) bereitgestellt: 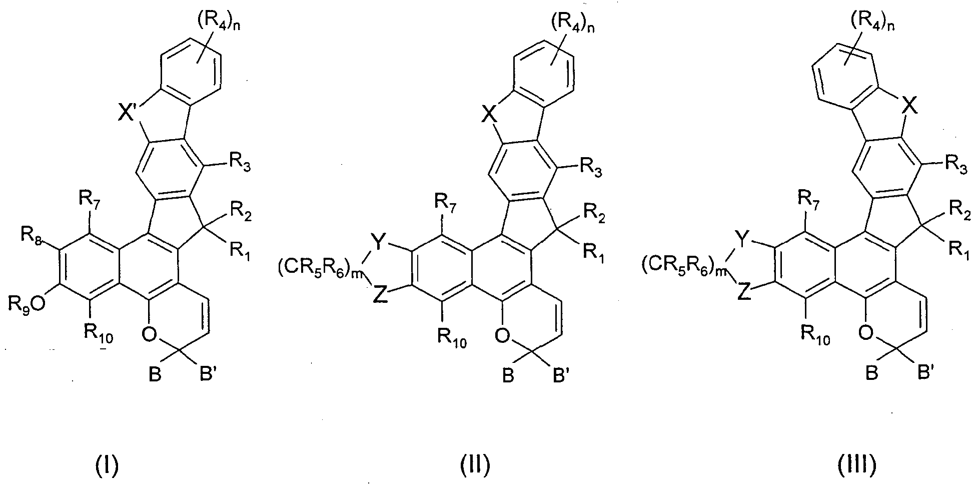
worin die Reste R₁, R₂, R₃, R₄, R₇, R₈ und R₁₀ jeweils unabhängig voneinander einen Substituenten darstellen, ausgewählt aus der Gruppe a, bestehend aus einem Wasserstoffatom, einem (C₁-C₁₈)-Alkyl-Rest, einem (C₃-C₇)-Cycloalkyl-Rest, der ein oder mehrere Heteroatome, wie beispielsweise O oder S, aufweisen kann, einem (C₁-C₁₈)-Thioalkyl-Rest, einem (C₁-C₁₈)-Alkoxy-Rest, einer Hydroxygruppe, einer Trifluormethylgruppe, Brom, Chlor, Fluor, einem un-, mono- oder disubstituiertem Phenyl-, Phenoxy-, Benzyl-, Benzyloxy-, Naphthyl- oder Naphthoxy-Rest, wobei diese Substituenten wiederum aus der Gruppe β ausgewählt sein können, bestehend aus einem Wasserstoffatom, einem (C₁-C₆)-Alkyl-Rest, einem (C₃-C₇)-Cycloalkyl-Rest, einem (C₁-C₆)-Thioalkyl-Rest, einem (C₁-C₆)-Alkoxy-Rest, einem Phenyl-Rest, einem Benzyl-Rest oder einem Naphthyl-Rest;
n dabei eine ganze Zahl von 1 bis 4 darstellt;
oder die Reste R₁ und R₂ zusammen mit dem an diese Reste gebundenen Kohlenstoffatom einen drei- bis achtgliedrigen carbo- oder heteromonocyclischen Ring bildet, der gegebenfalls einen oder mehrere Substituenten aus der Gruppe β trägt, und an den ein bis drei aromatische oder heteroaromatische Ringsysteme annelliert sein können, wobei das bzw. die Ringsysteme unabhängig voneinander aus Benzol, Naphthalin, Phenanthren, Pyridin, Chinolin, Furan, Thiophen, Pyrrol, Benzofuran, Benzothiophen, Indol und Carbazol ausgewählt sind, die wiederum mit einem oder mehreren Substituenten, ausgewählt aus der Gruppe β, substituiert sein können;
oder zwei benachbarte Reste R₄ einen annellierten Benzol-Ring bilden, der un-, mono- oder disubstituiert sein kann, wobei die Substituenten aus der Gruppe β ausgewählt sein können;
oder zwei benachbarte Reste R₄ ein über die Doppelbindung im Fünfring annelliertes 1,1-Dimethylinden- oder Benzofuran-Ringsystem bilden;
oder zwei benachbarte Reste R₄ ein über die Doppelbindung im heterocyclischen Sechsring annelliertes 2H-Chromen- oder 1H-Isochromen-Ringsystem bilden,
die Reste R₅ und R₆ jeweils unabhängig voneinander einen Substituenten darstellen, ausgewählt aus Wasserstoff, einem (C₁-C₁₈)-Alkyl-Rest, einem Phenyl-Rest, einem Benzyl-Rest oder einem Naphthyl-Rest;
m eine ganze Zahl von 1 bis 4, vorzugsweise 1 oder 2, mehr bevorzugt 2, darstellt;
oder zwei benachbarte -CR₅R₆-Gruppierungen einen annellierten Benzol-Ring bilden, der un-, mono- oder disubstituiert sein kann, wobei die Substituenten aus der Gruppe β ausgewählt sein können;
der Rest R₉ einen Substituenten darstellt, ausgewählt aus Wasserstoff, einem (C₁-C₁₈)-Alkyl-Rest, einem Phenyl-Rest, einem Benzyl-Rest oder einem Naphthyl-Rest;
X' in der Formel (I) ausgewählt ist aus -SO₂-, -CH₂-, -C(CH₃)₂-, -C(C₂H₅)₂-, -C(C₆H₅)₂-, -O-CH₂-, -CH₂-O-, -CH₂-CH₂-, -C(CH₃)₂-CH₂- oder -CH₂-C(CH₃)₂-;
X in der Formel (II) bzw. (III) ausgewählt ist aus -O-, -S-, -SO₂-, -N(C₁-C₆)-Alkyl-, -NC₆H₅-, -CH₂-, -C(CH₃)₂-, -C(C₂H₅)₂-, -C(C₆H₅)₂-, -O-CH₂-, -CH₂-O-, -O-(C=O)-, -(C=O)-O-, -CH₂-CH₂-, -C(CH₃)₂-CH₂- oder -CH₂-C(CH₃)₂-;
Y und Z unabhängig voneinander ausgewählt sind aus -O-, -S-, -SO₂-, -N(C₁-C₆)-Alkyl-, -NC₆H₅-, -CH₂-, -C(CH₃)₂-, -C(C₂H₅)₂- oder -C(C₆H₅)₂-, wobei Y oder Z gegebenenfalls zusammen mit der jeweils benachbarten CR₅R₆-Gruppierung einen annellierten Benzol-Ring bilden können, der un-, mono- oder disubstituiert sein kann, wobei die Substituenten wiederum aus der Gruppe β ausgewählt sein können, oder Y oder Z zusammen mit der jeweils benachbarten CR₅R₆-Gruppierung ein annelliertes Naphthalin-, 9,9-Dimethylfluoren- oder Dibenzofuran-Ringsystem bilden, die wiederum mit einem oder mehreren Substituenten, ausgewählt aus der Gruppe β, substituiert sein können;
und worin B und B' unabhängig voneinander aus einer der folgenden Gruppen a) oder b) ausgewählt ist, wobei
   a) ein mono-, di- und trisubstituierter Aryl-Rest ist, wobei der Aryl-Rest Phenyl, Naphthyl oder Phenanthryl ist, und
   b) ein un-, mono- und disubstituierter Heteroaryl-Rest ist, wobei der Heteroaryl-Rest aus Pyridyl, Furanyl, Thienyl, Benzofuranyl, Benzothienyl, 1,2,3,4-Tetrahydrocarbazolyl oder Julolidinyl ausgewählt ist;
   wobei die Substituenten der Aryl- und Heteroaryl-Reste in a) und b) ausgewählt sind aus der Gruppe β oder der Gruppierung -(O-CHR₁₃-CH₂)_{q}-OR₁₄, wobei R₁₃ Wasserstoff oder einen Methyl-Rest darstellt, R₁₄ Wasserstoff oder einen (C₁-C₆)-Alkyl-Rest darstellt und q eine ganze Zahl von 1 bis 50 darstellt;
   oder die Substituenten in a) und b) aus der Gruppe χ ausgewählt sind, bestehend aus Amino, Mono-(C₁-C₁₈)-alkylamino, Di-(C₁-C₁₈)-alkylamino, am Phenylring un-, mono- oder disubstituiertem Phenethenyl, un-, mono- oder disubstituiertem (Phenyl-imino)methylen, un-, mono- oder disubstituiertem (Phenylmethylen)imino und un-, mono- oder disubstituiertem Mono- und Diphenylamino, Piperidinyl, 3,5-Dimethylpiperidinyl, N-substituiertem Piperazinyl, Pyrrolidinyl, Imidazolidinyl, Pyrazolidinyl, Indolinyl, Morpholinyl, 2,6-Dimethylmorpholinyl, Thiomorpholinyl, Azacycloheptyl, Azacyclooctyl, un-, mono- oder disubstituiertem Phenothiazinyl, un-, mono- oder disubstituiertem Phenoxazinyl, un-, mono-, di- oder trisubstituiertem 9,10-Dihydroacridinyl, un-, mono- oder disubstituiertem 1,2,3,4-Tetrahydrochinolinyl, un-, mono- oder disubstituiertem 2,3-Dihydro-1,4-benzoxazinyl, un-, mono- oder disubstituiertem 1,2;3,4-Tetrahydroisochinolinyl, un-, mono- oder disubstituiertem Phenazinyl, un-, mono- oder disubstituiertem Carbazolyl, un-, mono- oder disubstituiertem 1,2,3,4-Tetrahydrocarbazolyl und un-, mono- oder disubstituiertem 10,11-Dihydrodibenz[b,f]azepinyl, wobei der oder die Substituenten unabhängig voneinander wiederum aus der Gruppe β ausgewählt sein können;
oder zwei direkt benachbarte Substituenten der Aryl- und Heteroaryl-Reste in a) und b) eine V-(CR₁₁R₁₂)ₚ-W-Gruppierung darstellen, wobei p eine ganze Zahl von 1 bis 3 darstellt, die Reste R₁₁ und R₁₂ jeweils unabhängig voneinander einen Substituenten, ausgewählt aus der Gruppe β, darstellen sowie V und W unabhängig voneinander aus -O-, -S-, -N(C₁-C₆)-Alkyl-, -NC₆H₅-, -CH₂-, -C(CH₃)₂-, -C(C₂H₅)₂- oder -C(C₆H₅)₂- ausgewählt sind;
oder zwei oder mehrere benachbarte CR₁₁R₁₂-Gruppierungen Teil eines annellierten Benzol-Ringes sind, der un-, mono- oder disubstituiert sein kann, wobei die Substituenten wiederum aus der Gruppe β ausgewählt sein können;
oder V und/oder W zusammen mit der jeweils benachbarten CR₁₁R₁₂-Gruppierung einen annellierten Benzol-Ring bilden, der un-, mono- oder disubstituiert sein kann, wobei die Substituenten wiederum aus der Gruppe β ausgewählt sein können.

Die erfindungsgemäßen Verbindungen zeichnen sich dadurch aus, dass an eine Indenonaphthopyran-Kernstruktur mindestens zwei weitere Ringsysteme an die zum Substituenten R₃ benachbarte C-C-Bindung des Benzolringes annelliert sind. Als Folge davon wird die längstwellige Absorptionsbande der geschlossenen Form dieser photochromen Farbstoffe so weit bathochrom verschoben, dass sie bis weit in den violetten bzw. blauen sichtbaren Wellenlängenbereich hineinreicht. Für den Übergang in die angeregte (farbige) Form benötigen die erfindungsgemäßen Verbindungen dadurch im Gegensatz zu herkömmlichen, im Stand der Technik verfügbaren photochromen Farbstoffen kein oder kaum UV-Licht mehr und dunkeln daher z.B. auch hinter einer Auto-Windschutzscheibe ein, die UV-Licht komplett absorbiert. Somit lassen sich beispielsweise Autofahrer-Brillen realisieren, die auch im geschlossenen Auto tief eindunkeln, wenn die Sonne direkt durch die komplett UV-absorbierende Windschutzscheibe strahlt.

Die erfindungsgemäßen Verbindungen zeichnen sich darüber hinaus durch ihre hervorragende UV-Härtungskompatibilität aus, d.h. sie überstehen - eingebracht z.B. in eine Acrylatmonomer-Matrix mit UV-Initiator - unbeschadet eine durch starkes UV-Licht ausgelöste radikalische Polymerisation der Matrix. Außerdem weisen sie bei sehr hoher Leistung eine sehr gute Lebensdauer aus. Sie sind in Kunststoffen aller Art einsetzbar.

Der Molekülstruktur der erfindungsgemäßen Verbindungen liegt eine Indenonaphthopyran-Kernstruktur zugrunde (jeweils mit den Substituenten R₁, R₂, R₃, R₇ und R₁₀ in den Formeln), welche die photolabile Pyran-Einheit (mit den Substituenten B und B') enthält. Diese ist für den photochromen Charakter verantwortlich, da durch Lichtanregung die Bindung zwischen dem Sauerstoff der Pyran-Einheit und dem Kohlenstoffatom mit den Substituenten B und B' reversibel gebrochen wird, wodurch ein farbiges Merocyanin-System entsteht.

Wie vorstehend bereits ausgeführt, liegt der vorliegenden Erfindung die überraschende Feststellung zugrunde, dass bei einer Mehrfach-Annellierung an die Indenonaphthopyran-Kernstruktur die längstwellige Absorptionsbande der geschlossenen Form der erfindungsgemäßen photochromen Verbindungen so weit bathochrom verschoben werden kann, dass sie bis weit in den violetten bzw. blauen sichtbaren Wellenlängenbereich hineinreicht. Dieser Umstand konnte auf zwei Wegen erreicht werden.

Zum einen werden Verbindungen gemäß der Formel (I) bereitgestellt, die eine Annellierung von mindestens zwei weiteren Ringsystemen (mit X' sowie den Substituenten R₄) an die zum Substituenten R₃ benachbarte C-C-Bindung des Benzolringes aufweisen. Mit X' spezifisch ausgewählt aus -SO₂-, -CH₂-, -C(CH₃)₂-, -C(C₂H₅)₂-, -C(C₆H₅)₂-, -O-CH₂-, -CH₂-O-, -CH₂-CH₂-, -C(CH₃)₂-CH₂- oder -CH₂-C(CH₃)₂- ist in den Verbindungen gemäß der Formel (I) - im Vergleich zum Stand der Technik aus EP 0 912 908 - die längstwellige Absorptionsbande der geschlossenen Form deutlich bathochrom verschoben.

Zum anderen werden Verbindungen gemäß den Formeln (II) und (III) bereitgestellt, die darüber hinaus eine weitere Annellierung mindestens eines Ringsystems (mit Y und Z sowie den Substituenten R₅ und R₆) an die C-C-Bindung zwischen den Substituenten R₇ und R₁₀ aufweisen. Diese mehrfach-annellierten Indenonaphthopyrane weisen extrem langwellige und intensive Absorptionsbanden der geschlossenen Form auf.

Die langwellige Absorption der geschlossenen Form der erfindungsgemäßen Verbindungen bis weit in den sichtbaren Wellenlängenbereich hinein bringt naturgemäß eine gelbe Vorfärbung dieser photochromen Farbstoffe mit sich. Um diese Vorfärbung in den Verwendungen zu kaschieren, können je nach Belieben violette oder blaue Permanentfarbstoffe, wie sie im Stand der Technik bekannt sind, zugesetzt werden, die zusammen mit den erfindungsgemäßen Verbindungen dann neutral graue Vorfärbungen ergeben.

In einer Ausführungsform der vorliegenden Erfindung sind die Reste R₁, R₂, R₃, R₄, R₇, R₈ und R₁₀ unabhängig voneinander aus einem Wasserstoffatom, einem (C₁-C₆)-Alkylrest oder einem (C₃-C₇)-Cycloalkylrest ausgewählt.

In einer weiteren Ausführungsform der vorliegenden Erfindung ist X' in der Formel (I) aus -CH₂-, -C(CH₃)₂-, -C(C₂H₅)₂-, -C(C₆H₅)₂-, -O-CH₂- oder -CH₂-O- ausgewählt.

In einer anderen Ausführungsform der vorliegenden Erfindung ist X in der Formel (II) bzw. (III) aus -O-, -S-, -CH₂-, -C(CH₃)₂-, -C(C₂H₅)₂-, -C(C₆H₅)₂-, -O-CH₂-, -CH₂-O-, -CH₂-CH₂-, -C(CH₃)₂-CH₂- oder -CH₂-C(CH₃)₂- ausgewählt.

In einer weiteren Ausführungsform der vorliegenden Erfindung sind Y und Z unabhängig voneinander aus -O-, -CH₂-, -C(CH₃)₂-, -C(C₂H₅)₂- oder -C(C₆H₅)₂-ausgewählt, wobei Y oder Z wiederum gegebenenfalls zusammen mit der jeweils benachbarten CR₅R₆-Gruppierung einen annellierten Benzol-Ring bilden können, der un-, mono- oder disubstituiert sein kann, wobei die Substituenten wiederum aus der Gruppe β ausgewählt sein können.

Der Rest R₉ ist vorzugsweise ein (C₁-C₁₈)-Alkyl-Rest, ein Phenyl-Rest oder ein Benzyl-Rest.

In einer bevorzugten Ausführungsform der vorliegenden Erfindung sind die Reste B und B' unabhängig voneinander aus der Gruppe a), wie vorstehend definiert, ausgewählt.

Unabhängig von dem Vorstehenden weisen in einer Ausführungsform der vorliegenden Erfindung die erfindungsgemäßen photochromen mehrfach-annellierten Naphthopyrane die allgemeine Formel (I) auf.

Zur Messung der spektralen und photochromen Eigenschaften der erfindungsgemäßen Verbindungen wurden jeweils 350 ppm des photochromen Farbstoffes in einer Acrylatmonomer-Matrix gelöst und nach Zusatz eines Polymerisations-Initiators mit Hilfe eines Temperaturprogrammes thermisch polymerisiert.

Die photochromen Eindunklungs-Eigenschaften ("Sättigungs-Absorption der offenen Form"; siehe Tab.1) der so hergestellten Probekörper wurden anschließend gemäß DIN EN ISO 8980-3 ermittelt.

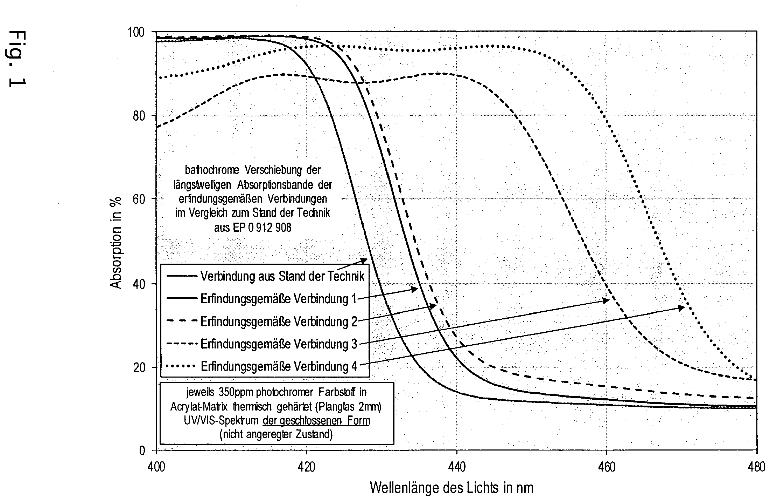

Figur 1 zeigt die Absorptionsspektren (400-480 nm) erfindungsgemäßer Verbindungen in der geschlossenen Form (nicht angeregter Zustand) im Vergleich zum Stand der Technik aus EP 0 912 908.

Die Molekülstruktur der in der Figur 1 dargestellten Verbindungen sind in der nachstehenden Tabelle 1 aufgeführt. Die erfindungsgemäßen Verbindungen 1 und 2 werden durch die nachstehende Formel (IV) beschrieben, die erfindungsgemäße Verbindung 3 durch die nachstehende Formel (VI) sowie die erfindungsgemäße Verbindung 4 durch die nachstehende Formel (V).

Die längstwellige Absorptionsbande der geschlossenen Form der erfindungsgemäßen Verbindungen ist im Vergleich zum Stand der Technik signifikant zu längeren Wellenlängen hin verschoben. Dadurch dunkeln die erfindungsgemäßen Verbindungen auch noch gut hinter einer komplett UV-absorbierenden Auto-Windschutzscheibe ein (siehe Tabelle 1).

Um dies zu quantifizieren, wurden die Messungen der Eindunklungs-Eigenschaften einmal ohne ("Standard") und einmal mit vorgeschaltetem Langpass-Filter durchgeführt (siehe Tabelle 1). Der verwendete Langpass-Filter (GG400; Fa. Schott) absorbiert den UV-Anteil des Anregungslichtes vollständig, wodurch eine gute Simulation der Verhältnisse hinter einer Auto-Windschutzscheibe erreicht wird.

**Tabelle 1: Vergleich der Sättigungsabsorptionen der offenen Formen (angeregter Zustand):**

| | | | | | |
|---|---|---|---|---|---|
| Standard-Messung gemäß DIN EN ISO 8980-3 vs. Messung mit GG400-Filter (An = Anisyl, d.h. 4-Methoxyphenyl-Rest) | | | | | |
| 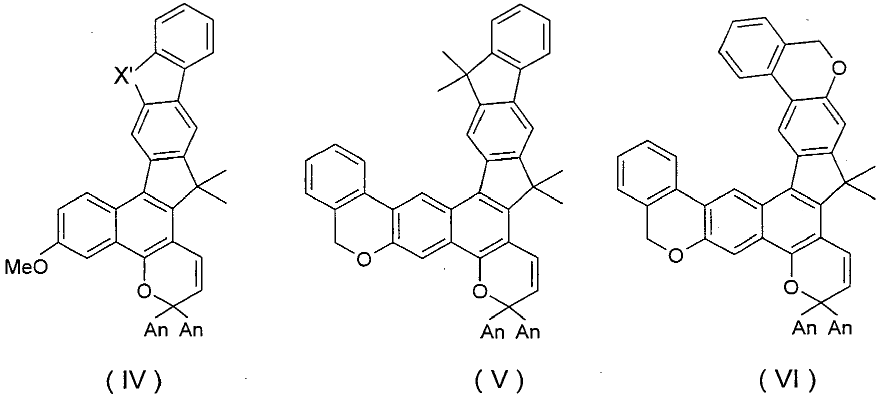 | | | | | |

| | X' in Formel (IV) | Wellenlänge mit 50% Absorption (geschlossene Form; s. Figur 1) | Sättigungs-absorption 23°C (offene Form) Standard-Messung | Sättigungs-absorption 23°C (offene Form) Messung mit GG400-Filter | Differenz der Sättigungsabsorptionen (Standard - GG400) |
|---|---|---|---|---|---|
| Stand der Technik aus EP 0 912 908 (Formel (IV)) | S | 428 nm | 88% | 77% | 11% |
| Erfindungsgemäße Verbindung 1 (Formel (IV)) | CMe₂ | 433 nm | 87% | 81% | 6% |
| Erfindungsgermäße Verbindung 2 (Formel (IV)) | 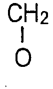 | 435 nm | 87% | 82% | 5% |
| Erfindungsgemäße Verbindung 3 (Formel ( VI )) | | 457 nm | 84% | 80% | 4% |
| Erfindungsgemäße Verbindung 4 (Formel (V)) | | 467 nm | 86% | 84% | 2% |

Die Differenz der Sättigungs-Absorptionen zwischen der Standard-Messung (ohne GG400-Filter) und der Messung mit GG400-Filter ist eine aussagekräftige Kenngröße. Je kleiner diese Differenz ist, umso weniger UV-Licht unterhalb von 400 nm wird für die Eindunklung benötigt und umso mehr eignet sich der photochrome Farbstoff zum Einsatz in einer Autofahrer-Brille. Die erfindungsgemäßen Verbindungen weisen alle einen deutlich geringeren Differenzwert im Vergleich zu Verbindungen des Standes der Technik auf und benötigen für die Eindunklung praktisch kein UV-Licht unterhalb von 400 nm.

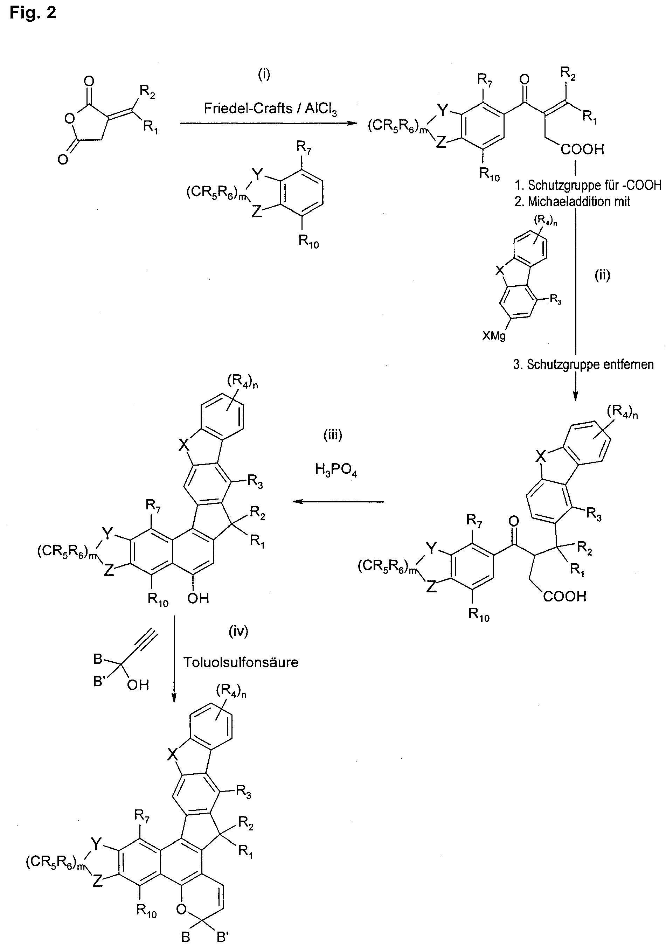

Zur Synthese der erfindungsgemäßen Verbindungen (siehe Syntheseschema gemäß der Figur 2) werden geeignet substituierte Methylidensuccinanhydride in einem ersten Schritt einer Friedel-Crafts-Reaktion mit geeignet substituierten aromatischen Systemen unterworfen (Schritt (i)). Die -COOH Gruppe des daraus resultierenden Intermediats wird anschließend geschützt und dieses Intermediat einer Michael-Addition mit entsprechend substituierten Aryl-Grignardverbindungen unterworfen (Schritt (ii)). Nach Entfernung der Carbonsäure-Schutzgruppe werden via intramolekularer Cyclisierung mittels Phosphorsäure entsprechend substituierte Naphthol-Derivate gebildet (Schritt (iii)). Anschließend werden diese mit geeignet substituierten 2-Propin-1-ol-Derivaten gemäß Schritt (iv) zu den erfindungsgemäßen Verbindungen umgesetzt.

Die erfindungsgemäßen Verbindungen können in Kunststoffmaterialien bzw. Kunststoffgegenständen jeglicher Art und Form für eine Vielzahl von Einsatzzwecken, für die photochromes Verhalten von Bedeutung ist, verwendet werden. Dabei können ein Farbstoff gemäß der vorliegenden Erfindung oder ein Gemisch solcher Farbstoffe eingesetzt werden. Beispielsweise können die erfindungsgemäßen photochromen Naphthopyran-Farbstoffe in Linsen, insbesondere ophthalmischen Linsen, Gläsern für Brillen aller Art, wie beispielsweise Skibrillen, Sonnenbrillen, Motorradbrillen, Visieren von Schutzhelmen und dergleichen, insbesondere in Autofahrer-Brillen eingesetzt werden. Ferner können die erfindungsgemäßen photochromen Naphthopyrane beispielsweise auch als Sonnenschutz in Fahrzeugen und Wohnräumen in Form von Fenstern, Schutzblenden, Abdeckungen, Dächern oder dergleichen verwendet werden.

Zur Herstellung von solchen photochromen Gegenständen können die erfindungsgemäßen photochromen Naphthopyrane durch verschiedene, im Stand der Technik beschriebene Verfahren, wie bereits in WO 99/15518 angegeben, auf ein Polymermaterial, wie ein organisches Kunststoffmaterial, aufgebracht oder darin eingebettet werden.

Es werden dabei sogenannte Massefärbungs- und Oberflächenfärbungsverfahren unterschieden. Ein Massefärbungsverfahren umfasst beispielsweise das Auflösen oder Dispergieren der photochromen Verbindung oder Verbindungen gemäß der vorliegenden Erfindung in einem Kunststoffmaterial, z.B. durch die Zugabe der photochromen Verbindung(en) zu einem monomeren Material, bevor die Polymerisation erfolgt. Eine weitere Möglichkeit zur Herstellung eines photochromen Gegenstands ist die Durchdringung des oder der Kunststoffmaterialien mit der (den) photochromen Verbindung(en) durch Eintauchen des Kunststoffmaterials in eine heiße Lösung des oder der photochromen Farbstoffe gemäß der vorliegenden Erfindung oder beispielsweise auch ein Thermotransferverfahren. Die photochrome(n) Verbindung(en) kann bzw. können beispielsweise auch in Form einer separaten Schicht zwischen aneinandergrenzenden Schichten des Kunststoffmaterials, z.B. als Teil eines polymeren Films, vorgesehen werden. Ferner ist auch ein Aufbringen der photochromen Verbindung(en) als Teil einer auf der Oberfläche des Kunststoffmaterials befindlichen Beschichtung möglich. Der Ausdruck "Durchdringung" soll dabei die Migration der photochromen Verbindung(en) in das Kunststoffmaterial, z.B. durch den lösungsmittelunterstützten Transfer der photochromen Verbindung(en) in eine Polymermatrix, Dampfphasentransfer oder andere derartige Oberflächendiffusionsvorgänge, bedeuten. Vorteilhafterweise können solche photochromen Gegenstände, wie z.B. Brillengläser, nicht nur mittels der üblichen Massefärbung, sondern in gleicher Weise auch mittels Oberflächenfärbung hergestellt werden, wobei bei der letzteren Variante eine überraschend geringere Migrationsneigung erzielt werden kann. Dies ist vor allem bei nachfolgenden Veredelungsschritten von Vorteil, da - z.B. bei einer Antireflexbeschichtung durch die geringere Rückdiffusion im Vakuum - Schichtablösungen und ähnliche Defekte drastisch verringert werden.

Insgesamt können auf Basis der erfindungsgemäßen photochromen Naphthopyrane beliebig kompatible (in chemischer Hinsicht und farblicher Art und Weise verträgliche) Färbungen, d.h. Farbstoffe, auf das Kunststoffmaterial aufgebracht oder in es eingebettet werden, um sowohl ästhetischen Gesichtspunkten als auch medizinischen oder modischen Aspekten zu genügen. Der oder die spezifisch ausgewählte(n) Farbstoff(e) kann bzw. können demzufolge, abhängig von den beabsichtigten Wirkungen sowie Anforderungen, variieren.

## Patentansprüche

1. Photochrome mehrfach-annellierte Naphthopyrane mit den Formeln (I), (II) bzw. (III): 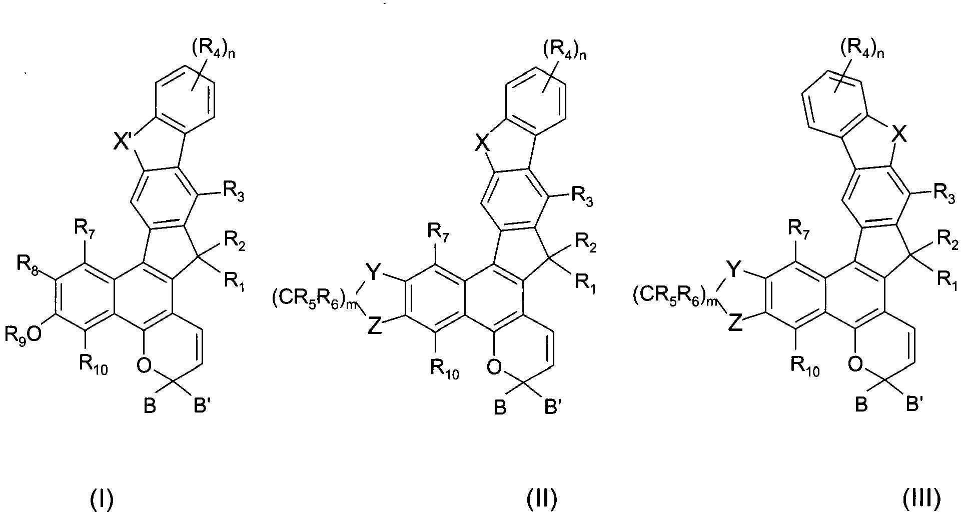
worin die Reste R₁, R₂, R₃, R₄, R₇, R₈ und R₁₀ jeweils unabhängig voneinander einen Substituenten darstellen, ausgewählt aus der Gruppe a, bestehend aus einem Wasserstoffatom, einem (C₁-C₁₈)-Alkyl-Rest, einem (C₃-C₇)-Cycloalkyl-Rest, der ein oder mehrere Heteroatome, wie beispielsweise O oder S, aufweisen kann, einem (C₁-C₁₈)-Thioalkyl-Rest, einem (C₁-C₁₈)-Alkoxy-Rest, einer Hydroxygruppe, einer Trifluormethylgruppe, Brom, Chlor, Fluor, einem un-, mono- oder disubstituiertem Phenyl-, Phenoxy-, Benzyl-, Benzyloxy-, Naphthyl- oder Naphthoxy-Rest, wobei diese Substituenten wiederum aus der Gruppe β ausgewählt sein können, bestehend aus einem Wasserstoffatom, einem (C₁-C₆)-Alkyl-Rest, einem (C₃-C₇)-Cycloalkyl-Rest, einem (C₁-C₆)-Thioalkyl-Rest, einem (C₁-C₆)-Alkoxy-Rest, einem Phenyl-Rest, einem Benzyl-Rest oder einem Naphthyl-Rest;
n dabei eine ganze Zahl von 1 bis 4 darstellt;
oder die Reste R₁ und R₂ zusammen mit dem an diese Reste gebundenen Kohlenstoffatom einen drei- bis achtgliedrigen carbo- oder heteromonocyclischen Ring bildet, der gegebenfalls einen oder mehrere Substituenten aus der Gruppe β trägt, und an den ein bis drei aromatische oder heteroaromatische Ringsysteme annelliert sein können, wobei das bzw. die Ringsysteme unabhängig voneinander aus Benzol, Naphthalin, Phenanthren, Pyridin, Chinolin, Furan, Thiophen, Pyrrol, Benzofuran, Benzothiophen, Indol und Carbazol ausgewählt sind, die wiederum mit einem oder mehreren Substituenten, ausgewählt aus der Gruppe β, substituiert sein können;
oder zwei benachbarte Reste R₄ einen annellierten Benzol-Ring bilden, der un-, mono- oder disubstituiert sein kann, wobei die Substituenten aus der Gruppe β ausgewählt sein können;
oder zwei benachbarte Reste R₄ ein über die Doppelbindung im Fünfring annelliertes 1,1-Dimethylinden- oder Benzofuran-Ringsystem bilden;
oder zwei benachbarte Reste R₄ ein über die Doppelbindung im heterocyclischen Sechsring annelliertes 2H-Chromen- oder 1H-Isochromen-Ringsystem bilden,
die Reste R₅ und R₆ jeweils unabhängig voneinander einen Substituenten darstellen, ausgewählt aus Wasserstoff, einem (C₁-C₁₈)-Alkyl-Rest, einem Phenyl-Rest, einem Benzyl-Rest oder einem Naphthyl-Rest;
m eine ganze Zahl von 1 bis 4, vorzugsweise 1 oder 2, mehr bevorzugt 2, darstellt;
oder zwei benachbarte -CR₅R₆-Gruppierungen einen annellierten Benzol-Ring bilden, der un-, mono- oder disubstituiert sein kann, wobei die Substituenten aus der Gruppe β ausgewählt sein können;
der Rest R₉ einen Substituenten darstellt, ausgewählt aus Wasserstoff, einem (C₁-C₁₈)-Alkyl-Rest, einem Phenyl-Rest, einem Benzyl-Rest oder einem Naphthyl-Rest;
X' in der Formel (I) ausgewählt ist aus -SO₂-, -CH₂-, -C(CH₃)₂-, -C(C₂H₅)₂-, -C(C₆H₅)₂-, -O-CH₂-, -CH₂-O-, -CH₂-CH₂-, -C(CH₃)₂-CH₂- oder -CH₂-C(CH₃)₂-;
X in der Formel (II) bzw. (III) ausgewählt ist aus -O-, -S-, -SO₂-, -N(C₁-C₆)-Alkyl-, - NC₆H₅-, -CH₂-, -C(CH₃)₂-, -C(C₂H₅)₂-, -C(C₆H₅)₂-, -O-CH₂-, -CH₂-O-, -O-(C=O)-, - (C=O)-O-, -CH₂-CH₂-, -C(CH₃)₂-CH₂- oder -CH₂-C(CH₃)₂-;
Y und Z unabhängig voneinander ausgewählt sind aus -O-, -S-, -SO₂-, -N(C₁-C₆)-Alkyl-, -NC₆H₅-, -CH₂-, -C(CH₃)₂-, -C(C₂H₅)₂- oder -C(C₆H₅)₂-, wobei Y oder Z gegebenenfalls zusammen mit der jeweils benachbarten CR₅R₆-Gruppierung einen annellierten Benzol-Ring bilden können, der un-, mono- oder disubstituiert sein kann, wobei die Substituenten wiederum aus der Gruppe β ausgewählt sein können, oder Y oder Z zusammen mit der jeweils benachbarten CR₅R₆-Gruppierung ein annelliertes Naphthalin-, 9,9-Dimethylfluoren- oder Dibenzofuran-Ringsystem bilden, die wiederum mit einem oder mehreren Substituenten, ausgewählt aus der Gruppe β, substituiert sein können;
und worin B und B' unabhängig voneinander aus einer der folgenden Gruppen a) oder b) ausgewählt ist, wobei
a) ein mono-, di- und trisubstituierter Aryl-Rest ist, wobei der Aryl-Rest Phenyl, Naphthyl oder Phenanthryl ist, und
b) ein un-, mono- und disubstituierter Heteroaryl-Rest ist, wobei der Heteroaryl-Rest aus Pyridyl, Furanyl, Thienyl, Benzofuranyl, Benzothienyl, 1,2,3,4-Tetrahydrocarbazolyl oder Julolidinyl ausgewählt ist;
wobei die Substituenten der Aryl- und Heteroaryl-Reste in a) und b) ausgewählt sind aus der Gruppe β oder der Gruppierung -(O-CHR₁₃-CH₂)_{q}-OR₁₄, wobei R₁₃ Wasserstoff oder einen Methyl-Rest darstellt, R₁₄ Wasserstoff oder einen (C₁-C₆)-Alkyl-Rest darstellt und q eine ganze Zahl von 1 bis 50 darstellt;
oder die Substituenten in a) und b) aus der Gruppe χ ausgewählt sind, bestehend aus Amino, Mono-(C₁-C₁₈)-alkylamino, Di-(C₁-C₁₈)-alkylamino, am Phenylring un-, mono- oder disubstituiertem Phenethenyl, un-, mono- oder disubstituiertem (Phenyl-imino)methylen, un-, mono- oder disubstituiertem (Phenylmethylen)imino und un-, mono- oder disubstituiertem Mono- und Diphenylamino, Piperidinyl, 3,5-Dimethylpiperidinyl, N-substituiertem Piperazinyl, Pyrrolidinyl, Imidazolidinyl, Pyrazolidinyl, Indolinyl, Morpholinyl, 2,6-Dimethylmorpholinyl, Thiomorpholinyl, Azacycloheptyl, Azacyclooctyl, un-, mono- oder disubstituiertem Phenothiazinyl, un-, mono- oder disubstituiertem Phenoxazinyl, un-, mono-, di- oder trisubstituiertem 9,10-Dihydroacridinyl, un-, mono- oder disubstituiertem 1,2,3,4-Tetrahydrochinolinyl, un-, mono- oder disubstituiertem 2,3-Dihydro-1,4-benzoxazinyl, un-, mono- oder disubstituiertem 1,2,3,4-Tetrahydroisochinolinyl, un-, mono- oder disubstituiertem Phenazinyl, un-, mono- oder disubstituiertem Carbazolyl, un-, mono- oder disubstituiertem 1,2,3,4-Tetrahydrocarbazolyl und un-, mono- oder disubstituiertem 10,11-Dihydrodibenz[b,f]azepinyl, wobei der oder die Substituenten unabhängig voneinander wiederum aus der Gruppe β ausgewählt sein können;
oder zwei direkt benachbarte Substituenten der Aryl- und Heteroaryl-Reste in a) und b) eine V-(CR₁₁R₁₂)ₚ-W-Gruppierung darstellen, wobei p eine ganze Zahl von 1 bis 3 darstellt, die Reste R₁₁ und R₁₂ jeweils unabhängig voneinander einen Substituenten, ausgewählt aus der Gruppe β, darstellen sowie V und W unabhängig voneinander aus -O-, -S-, -N(C₁-C₆)-Alkyl-, -NC₆H₅-, -CH₂-, -C(CH₃)₂-, -C(C₂H₅)₂- oder -C(C₆H₅)₂-ausgewählt sind;
oder zwei oder mehrere benachbarte CR₁₁R₁₂-Gruppierungen Teil eines annellierten Benzol-Ringes sind, der un-, mono- oder disubstituiert sein kann, wobei die Substituenten wiederum aus der Gruppe β ausgewählt sein können;
oder V und/oder W zusammen mit der jeweils benachbarten CR₁₁R₁₂-Gruppierung einen annellierten Benzol-Ring bilden, der un-, mono- oder disubstituiert sein kann, wobei die Substituenten wiederum aus der Gruppe β ausgewählt sein können.

2. Photochrome mehrfach-annellierte Naphthopyrane gemäß Anspruch 1, wobei die Reste R₁, R₂, R₃, R₄, R₇, R₈ und R₁₀ unabhängig voneinander aus einem Wasserstoffatom, einem (C₁-C₆)-Alkylrest oder einem (C₃-C₇)-Cycloalkylrest ausgewählt sind.

3. Photochrome mehrfach-annellierte Naphthopyrane gemäß Anspruch 1 oder 2, wobei X' in der Formel (I) aus -CH₂-, -C(CH₃)₂-, -C(C₂H₅)₂-, -C(C₆H₅)₂-, -O-CH₂- oder - CH₂-O- ausgewählt ist.

4. Photochrome mehrfach-annellierte Naphthopyrane gemäß Anspruch 1 oder 2, wobei X in der Formel (II) bzw. (III) aus -O-, -S-, -CH₂-, -C(CH₃)₂-, -C(C₂H₅)₂-, -C(C₆H₅)₂-, -O-CH₂-, -CH₂-O-, -CH₂-CH₂-, -C(CH₃)₂-CH₂- oder -CH₂-C(CH₃)₂- ausgewählt ist.

5. Photochrome mehrfach-annellierte Naphthopyrane gemäß Anspruch 1, 2 oder 4, wobei Y und Z unabhängig voneinander ausgewählt sind aus -O-, -CH₂-, -C(CH₃)₂-, -C(C₂H₅)₂- oder -C(C₆H₅)₂-, wobei Y oder Z gegebenenfalls zusammen mit der jeweils benachbarten CR₅R₆-Gruppierung einen annellierten Benzol-Ring bilden können, der un-, mono- oder disubstituiert sein kann, wobei die Substituenten wiederum aus der Gruppe β ausgewählt sein können.

6. Photochrome mehrfach-annellierte Naphthopyrane gemäß Anspruch 1, 2 oder 3, wobei der Rest R₉ ein (C₁-C₁₈)-Alkyl-Rest, ein Phenyl-Rest oder ein Benzyl-Rest ist.

7. Photochrome mehrfach-annellierte Naphthopyrane gemäß einem der vorhergehenden Ansprüche, wobei die Reste B und B' unabhängig voneinander aus der Gruppe a), wie vorstehend definiert, ausgewählt sind.

8. Photochrome mehrfach-annellierte Naphthopyrane gemäß einem der vorhergehenden Ansprüche, welche die allgemeine Formel (I) aufweisen.

9. Photochrome mehrfach-annellierte Naphthopyrane gemäß einem der vorhergehenden Ansprüche, welche die allgemeine Formel (IV), (V) bzw. (VI) aufweisen, 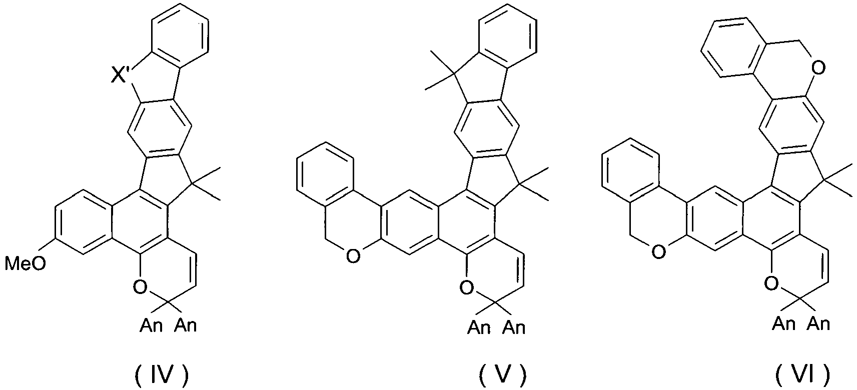 wobei X' bzw. X wie vorstehend definiert sind und An für Anisyl steht.

10. Verwendung der photochromen mehrfach-annellierten Naphthopyrane gemäß einem der Ansprüche 1 bis 9 in und auf Kunststoffmaterialien.

11. Verwendung gemäß Anspruch 10, wobei das Kunststoffmaterial eine ophthalmische Linse ist, insbesondere für Autofahrer-Brillen.

## Claims

1. Photochromic multi-anellated naphthopyrans with the formulas (I), (II) or (III): 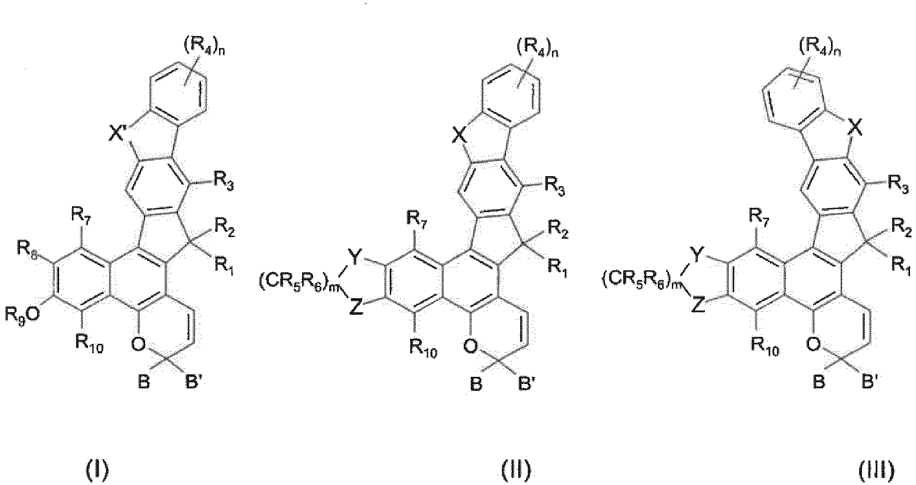
wherein the radicals R₁, R₂, R₃, R₄, R₇, R₈ and R₁₀ each independently represent a substituent selected from the group a, consisting of a hydrogen atom, a (C₁-C₁₈) alkyl radical, a (C₃-C₇) cycloalkyl radical, which can have one or more heteroatoms, such as O or S, a (C₁-C₁₈) thioalkyl radical, a (C₁-C₁₈) alkoxy radical, a hydroxy group, a trifluoromethyl group, bromine, chlorine, fluorine, an un-, mono- or disubstituted phenyl, phenoxy, benzyl, benzyloxy, naphthyl or naphthoxy radical, these substituents being selected from the group β, consisting of a hydrogen atom, a (C₁-C₆) alkyl radical, a (C₃-C₇) cycloalkyl radical, a (C₁-C₆) thioalkyl radical, a (C₁-C₆) alkoxy radical, a phenyl radical, a benzyl radical or a naphthyl radical;
n represents an integer from 1 to 4;
or the radicals R₁ and R₂ together with the carbon atom bonded to these radicals forms a three- to eight-membered carbo- or heteromonocyclic ring which optionally bears one or more substituents from the group β and to which one to three aromatic or heteroaromatic ring systems can be anellated, where the ring system or systems are selected independently from benzene, naphthalene, phenanthrene, pyridine, quinoline, furan, thiophene, pyrrole, benzofuran, benzothiophene, indole and carbazole, which may be substituted with one or more substituents selected from the group β;
or two adjacent radicals R₄ form an anellated benzene ring which can be unsubstituted, mono- or disubstituted, where the substituents can be selected from the group β;
or two adjacent radicals R₄ form a 1,1-dimethylindene or benzofuran ring system anellated via the double bond in the five-membered ring;
or two adjacent radicals R₄ form a 2H-chromene or 1 H-isochromene ring system anellated via the double bond in the heterocyclic six-membered ring,
the radicals R₅ and R₆ each independently represent a substituent selected from hydrogen and a (C₁-C₁₈) alkyl radical, a phenyl radical, a benzyl radical or a naphthyl radical;
m represents an integer from 1 to 4, preferably 1 or 2, more preferably 2;
or two adjacent -CR₅R₆ groups form a anellated benzene ring which can be un-, mono- or disubstituted, where the substituents are selected from the group β;
the radical R9 represents a substituent selected from hydrogen, a (C₁-C₁₈) alkyl radical, a phenyl radical, a benzyl radical or a naphthyl radical;
X' in formula (I) is selected from -SO₂-, -CH₂-, -C(CH₃)₂-, -C(C₂H₅)₂-, -C(C₆H₅)₂-, -O-CH₂-, - CH₂-O-, -CH₂-CH₂-, -C(CH₃)₂-CH₂- or -CH₂-C(CH₃)₂-;
X in formula (II) or (III) is selected from -O-, -S-, -SO₂-, -N(C1-C6)-Alkyl-, -NC₆H₅-, -CH₂-, - C(CH₃)₂-, -C(C₂H₅)₂-, -C(C₆H₅)₂-, -O-CH₂-, -CH₂-O-, -O-(C=O)-, -(C=O)-O-, -CH₂-CH₂-, - C(CH₃)₂-CH₂- or -CH₂-C(CH₃)₂-;
Y and Z are independently selected from -O-, -S-, -SO₂-, -N(C1-C6)-Alkyl-, -NC₆H₅-, -CH₂-, - C(CH₃)₂-, -C(C2H₅)₂- or -C(C₆H₅)₂-, where Y or Z, optionally together with the adjacent CR₅R₆ group, can form a anellated benzene ring which can be unsubstituted, mono- or disubstituted, where the substituents are selected from the group β, or Y or Z together with the adjacent CR₅R₆ group form a anellated naphthalene, 9,9-dimethylfluorene or dibenzofuran ring system, where one or more substituents can be substituted, selected from the group β;
and wherein B and B' are independently selected from one of the following groups a) or b), where
a) is a mono-, di- and trisubstituted aryl radical, the aryl radical being phenyl, naphthyl or phenanthryl, and
b) is an un-, mono- and disubstituted heteroaryl radical, the heteroaryl radical being selected from pyridyl, furanyl, thienyl, benzofuranyl, benzothienyl, 1,2,3,4-tetrahydrocarbazolyl or julolidinyl;
where the substituents of the aryl and heteroaryl radicals in a) and b) are selected from group β or the grouping -(O-CHR₁₃-CH₂)_{q}-OR₁₄, where R₁₃ represents hydrogen or a methyl radical, R₁₄ represents hydrogen or represents a (C₁-C₆) alkyl radical and q represents an integer from 1 to 50;
or the substituents in a) and b) are selected from the group χ, consisting of amino, mono-(C₁-C₁₈) -alkylamino, di-(C₁-C₁₈) -alkylamino, phenethenyl which is un-, mono- or disubstituted on the phenyl ring , un-, mono- or disubstituted (phenyl-imino) methylene, un-, mono- or disubstituted (phenylmethylene) imino and un-, mono- or disubstituted mono- and diphenylamino, piperidinyl, 3,5-dimethylpiperidinyl, N-substituted Piperazinyl, pyrrolidinyl, imidazolidinyl, pyrazolidinyl, indolinyl, morpholinyl, 2,6-dimethylmorpholinyl, thiomorpholinyl, azacycloheptyl, azacyclooctyl, un-, mono- or disubstituted phenothiazinyl, un-, mono- or disubstituted phenoxazinyl, un- or trisubstituted 9,10-dihydroacridinyl, un-, mono- or disubstituted 1,2,3,4-Tetrahydroquinolinyl, un-, mono- or disubstituted 2,3-dihydro-1,4-benzoxazinyl, un-, mono- or disubstituted 1,2,3,4-tetrahydroisoquinolinyl, un-, mono - or disubstituted phenazinyl, un-, mono- or disubstituted carbazolyl, un-, mono- or disubstituted 1, 2,3,4-tetrahydrocarbazolyl and un-, mono- or disubstituted 10,11-dihydrodibenz[b, f] azepinyl, where the substituent or substituents can in turn be selected independently of one another from the group β;
or two directly adjacent substituents of the aryl and heteroaryl radicals in a) and b) represent a V-(CR₁₁R₁₂)_{P}-W grouping, where p represents an integer from 1 to 3, the radicals R₁₁ and R₁₂ each independently of one another a substituent, selected from the group β, and V and W independently of one another from -O-, -S-, -N (C₁-C₆) -alkyl-, -NC₆H₅-, -CH₂-, -C(CH₃)₂-, - C(C₂H₅)₂- or -C(C₆H₅)₂-;
or two or more adjacent CR₁₁R₁₂ groups are part of an anellated benzene ring, which can be un-, mono- or disubstituted, the substituents in turn being selected from the group β;
or V and / or W together with the respectively adjacent CR₁₁R₁₂ group form an anellated benzene ring which can be unsubstituted, mono- or disubstituted, the substituents in turn being selected from the group β.

2. Photochromic multi-anellated naphthopyrans according to claim 1, wherein the radicals R₁, R₂, R₃, R₄, R₇, R₈ and R₁₀ are selected independently of one another from a hydrogen atom, a (C₁-C₆) alkyl radical or a (C₃-C₇) cycloalkyl radical

3. Photochromic multi-anellated naphthopyrans according to claim 1 or 2, wherein X' in the formula (I) is selected from -CH₂-, -C(CH₃)₂-, -C(C₂H₅)₂-, -C(C₆H₅)₂-, -O-CH₂- or -CH₂-O-.

4. Photochromic multi-anellated naphthopyrans according to claim 1 or 2, wherein X in the formula (II) or (III) is selected from -O-, -S-, -CH₂-, -C(CH₃)₂-, -C(C₂H₅)₂-, -C(C₆H₅)₂-, -O-CH₂-, -CH₂-O-, -CH₂-CH₂-, -C(CH₃)₂-CH₂- or -CH₂-C(CH₃)₂-.

5. Photochromic multi-anellated naphthopyrans according to claim 1, 2 or 4, wherein Y and Z are independently selected from -O-, -CH₂-, -C(CH₃)₂-, -C(C₂H₅)₂- or -C(C₆H₅)₂-, where Y or Z, optionally together with the respective adjacent CR₅R₆ group, can form a anellated benzene ring which can be un-, mono- or disubstituted, the substituents in turn being selected from the group β.

6. Photochromic multi-anellated naphthopyrans according to claim 1, 2 or 3, wherein the radical Rg is a (C₁-C₁₈) alkyl radical, a phenyl radical or a benzyl radical.

7. Photochromic multi-anellated naphthopyrans according to one of the preceding claims, wherein the radicals B and B 'are selected independently of one another from group a), as defined above.

8. Photochromic multi-anellated naphthopyrans according to one of the preceding claims, which have the general formula (I).

9. Photochromic multi-anellated naphthopyrans according to one of the preceding claims, which have the general formula (IV), (V) or (VI), 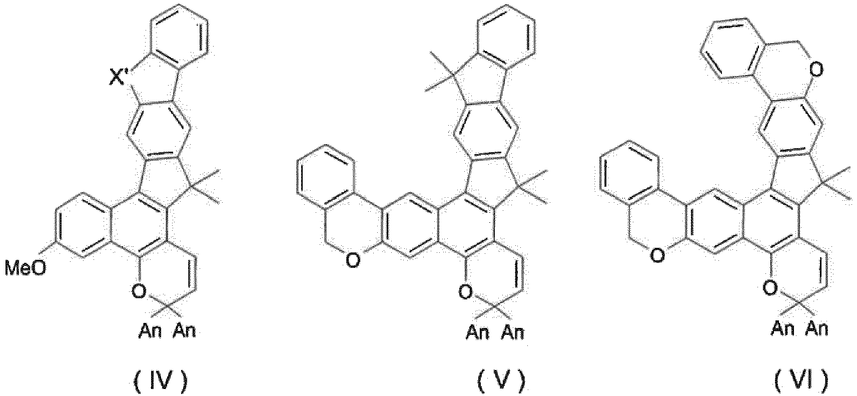 where X' and X are as defined above.

10. Use of the photochromic multi-anellated naphthopyrans according to one of claims 1 to 9 in and on plastic materials.

11. Use according to claim 10, wherein the plastic material is an ophthalmic lens, in particular for driving glasses.

## Revendications

1. Naphtopyranes photochromiques multi-condensés de formules (I), (II) ou (III): 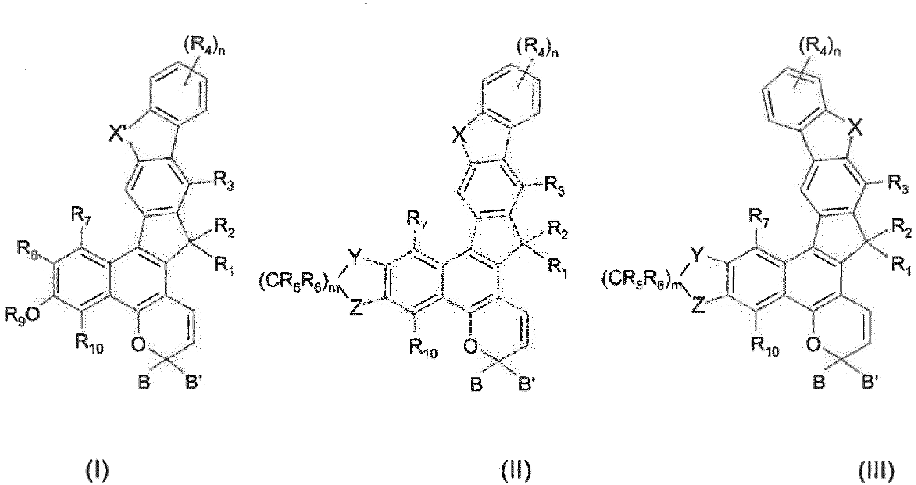
dans lesquels les radicaux R₁, R₂, R₃, R₄, R₇, R₈ et R₁₀ représentent chacun indépendamment un substituant choisi dans le groupe a, consistant en un atome d'hydrogène, un radical (C₁-C₁₈) alkyle, un (C₃-C₇) cycloalkyle, qui peut avoir un ou plusieurs hétéroatomes, tels que O ou S, un radical thioalkyle (C₁-C₁₈), un radical (C₁-C₁₈) alcoxy, un groupe hydroxy, un groupe trifluorométhyle, brome, chlore, fluor, un , un radical phényle, phénoxy, benzyle, benzyloxy, naphtyle ou naphtoxy mono- ou disubstitué, ces substituants étant choisis dans le groupe β, constitué par un atome d'hydrogène, un radical (C₁-C₆) alkyle, un (C₃-C₇) cycloalkyle , un radical (C₁-C₆) thioalkyle, un radical (C₁-C₆) alcoxy, un radical phényle, un radical benzyle ou un radical naphtyle;
n représente un entier de 1 à 4;
ou les radicaux R₁ et R₂ avec l'atome de carbone lié à ces radicaux forment un cycle carbo- ou hétéromonocyclique à trois à huit chaînons qui porte éventuellement un ou plusieurs substituants du groupe β et sur lequel un à trois systèmes cycliques aromatiques ou hétéroaromatiques peuvent être condensés, où le ou les systèmes cycliques sont choisis indépendamment parmi le benzène, le naphtalène, le phénanthrène, la pyridine, la quinoléine, le furane, le thiophène, le pyrrole, le benzofurane, le benzothiophène, l'indole et le carbazole, qui peuvent être substitués par un ou plusieurs substituants choisis parmi le groupe β;
ou deux radicaux adjacents R₄ forment un cycle benzénique condensé qui peut être non substitué, mono- ou disubstitué, les substituants pouvant être choisis dans le groupe β;
ou deux radicaux adjacents R₄ forment un système cyclique 1,1-diméthylindène ou benzofurane fusionné via la double liaison dans le cycle à cinq chaînons;
ou deux radicaux adjacents R₄ forment un système cyclique 2H-chromène ou 1 H-isochromène fusionné via la double liaison dans le cycle hétérocyclique à six chaînons, les radicaux R₅ et R₆ représentent chacun indépendamment un substituant choisi parmi l'hydrogène et un radical (C₁-C₁₈) alkyle, un radical phényle, un radical benzyle ou un radical naphtyle;
m représente un entier de 1 à 4, de préférence 1 ou 2, mieux encore 2;
ou deux groupes -CR₅R₆- adjacents forment un cycle benzène condensé qui peut être non substitué, mono- ou disubstitué, les substituants étant choisis dans le groupe β;
le radical Rg représente un substituant choisi parmi l'hydrogène, un radical (C₁-C₁₈) alkyle, un radical phényle, un radical benzyle ou un radical naphtyle; X 'dans la formule (I) est choisi parmi -SO₂-, -CH₂-, -C(CH₃)₂-, -C(C₂H₅)₂-, -C(C₆H₅)₂-, -O-CH₂-, -CH₂-O-, -CH₂-CH₂-, - C(CH₃)₂-CH₂- ou -CH₂-C(CH₃)₂-;
X dans la formule (II) ou (III) est choisi parmi -O-, -S-, -SO₂-, -N(C1-C6)-Alkyl-, -NC₆H₅-, - CH₂-, -C(CH₃)₂-, -C(C₂H₅)₂-, -C(C₆H₅)₂-, -O-CH₂-, -CH₂-O-, -O-(C=0)-, -(C=O)-O-, -CH₂-CH₂-, -C(CH₃)₂-CH₂- ou -CH₂-C(CH₃)₂-;
Y et Z sont choisis indépendamment parmi -O-, -S-, -SO₂-, -N(C1-C6)-Alkyl-, -NC₆H₅-, -CH₂-, -C(CH₃)₂-, -C(C₂H₅)₂- ou -C(C₆H₅)₂-, où Y ou Z, éventuellement conjointement avec le groupe CR₅R₆ adjacent, peuvent former un cycle benzène condensé qui peut être non substitué, mono- ou disubstitué, où les substituants sont choisis dans le groupe β, ou Y ou Z conjointement avec le groupe CR₅R₆ adjacent forment un système cyclique condensé naphtalène, 9,9-diméthylfluorène ou dibenzofurane, où un ou plusieurs substituants peuvent être substitués, choisis dans le groupe β;
et dans laquelle B et B 'sont choisis indépendamment parmi l'un des groupes a) ou b) suivants, où
a) est un radical aryle mono-, di- et trisubstitué, le radical aryle étant phényle, naphtyle ou phénanthryle, et
b) est un radical hétéroaryle non substitué, mono- et disubstitué, le radical hétéroaryle étant choisi parmi pyridyle, furanyle, thiényle, benzofuranyle, benzothiényle, 1,2,3,4-tétrahydrocarbazolyle ou julolidinyle;
où les substituants des radicaux aryle et hétéroaryle en a) et b) sont choisis dans le groupe β ou le groupement -(O-CHR₁₃-CH₂)_{q}-OR₁₄, où R₁₃ représente l'hydrogène ou un radical méthyle, R₁₄ représente l'hydrogène ou représente un (C₁-C₆) alkyle et q représente un entier de 1 à 50;
ou les substituants en a) et b) sont choisis dans le groupe χ, consistant en amino, mono- (C₁-C₁₈) -alkylamino, di- (C₁-C₁₈) -alkylamino, phénéthényle qui est non substitué, mono- ou disubstitué sur le cycle phényle, (phényl-imino) méthylène non substitué, mono- ou disubstitué, non substitué, mono- ou disubstitué (phénylméthylène) imino et mono- et diphénylamino non substitué, mono- ou disubstitué, pipéridinyle, 3,5-diméthylpipéridinyle , Pipérazinyle N-substitué, pyrrolidinyle, imidazolidinyle, pyrazolidinyle, indolinyle, morpholinyle, 2,6-diméthylmorpholinyl, thiomorpholinyle, azacycloheptyle, azacyclooctyle, phénothiazinyle non substitué, mono- ou disubstitué, phénothiazinyle non substitué, mono- ou disubstitué, phénothiazinyle non substitué, mono- ou disubstitué 9,10-dihydroacridinyle, non substitué, mono- ou disubstitué 1,2,3,4-tétrahydroquinolinyle, 2,3-dihydro-1,4-benzoxazinyle non, mono- ou disubstitué, non substitué, mono- ou disubstitué 1 , 2,3,4-tétrahydroisoquinolinyle, phénazinyle non substitué, mono ou disubstitué, carbazolyle non substitué, mono- ou disubstitué, non substitué, mono- ou disubstitué 1, 2, 3,4-tétrahydrocarbazolyle et 10,11-dihydrodibenz [b, f] azépinyle non substitué, mono- ou disubstitué, où le ou les substituants peuvent à leur tour être choisis indépendamment l'un de l'autre dans le groupe β;
ou deux substituants directement adjacents des radicaux aryle et hétéroaryle en a) et b) représentent un groupement V-(CR₁₁R₁₂)_{P}-W, où p représente un entier de 1 à 3, les radicaux R₁₁ et R₁₂ chacun indépendamment l'un de l'autre un substituant, choisis dans le groupe β, et V et W indépendamment l'un de l'autre parmi -O-, -S-, -N (C₁-C₆) -alkyl-, - NC₆H₅-, -CH₂-, -C(CH₃)₂-, -C(C₂H₅)₂- ou -C(C₆H₅)₂-;
ou deux ou plusieurs groupes CR₁₁R₁₂ adjacents font partie d'un cycle benzène condensé, qui peut être non substitué, mono- ou disubstitué, les substituants étant à leur tour choisis dans le groupe β;
ou V et / ou W conjointement avec le groupe CR₁₁R₁₂ respectivement adjacent forment un cycle benzène condensé qui peut être non substitué, mono- ou disubstitué, les substituants étant à leur tour choisis dans le groupe β.

2. Naphtopyranes photochromiques multi-condensés selon la revendication 1, dans lesquels les radicaux R₁, R₂, R₃, R₄, R₇, R₈ and R₁₀ sont choisis indépendamment l'un de l'autre parmi un atome d'hydrogène, un radical alkyle en (C₁-C₆) ou un radical cycloalkyle en (C₃-C₇).

3. Naphtopyranes photochromiques multi-condensés selon la revendication 1 ou 2, dans lesquels X' dans la formule (I) est choisi parmi -CH₂-, -C(CH₃)₂-, -C(C₂H₅)₂-, -C(C₆H₅)₂-, -O-CH₂- ou -CH₂-O-.

4. Naphtopyranes photochromiques multi-condensés selon la revendication 1 ou 2, dans lesquels X dans la formule (II) ou (III) est choisi parmi -O-, -S-, -CH₂-, -C(CH₃)₂-, -C(C₂H₅)₂-, - C(C₆H₅)₂-, -O-CH₂-, -CH₂-O-, -CH₂-CH₂-, -C(CH₃)₂-CH₂- ou -CH₂-C(CH₃)₂-.

5. Naphtopyranes photochromiques multi-condensés selon la revendication 1, 2 ou 4, dans lesquels Y et Z sont choisis indépendamment parmi -O-, -CH₂-, -C(CH₃)₂-, -C(C₂H₅)₂- ou - C(C₆H₅)₂-, où Y ou Z, éventuellement conjointement avec le groupe CR₅R₆ adjacent respectif, peuvent former un cycle benzène condensé qui peut être non substitué, mono- ou disubstitué, les substituants étant à leur tour choisis dans le groupe β.

6. Naphtopyranes photochromiques multi-condensés selon la revendication 1, 2 ou 3, dans lesquels le radical Rg est un radical (C₁-C₁₈) alkyle, un radical phényle ou un radical benzyle.

7. Naphtopyranes photochromiques multi-condensés selon l'une des revendications précédentes, dans lesquels les radicaux B et B 'sont choisis indépendamment l'un de l'autre dans le groupe a) tel que défini ci-dessus.

8. Naphtopyranes photochromiques multi-condensés selon l'une des revendications précédentes, répondant à la formule générale (I).

9. Naphtopyranes photochromiques multi-condensés selon l'une des revendications précédentes, répondant à la formule générale (IV), (V) ou (VI), 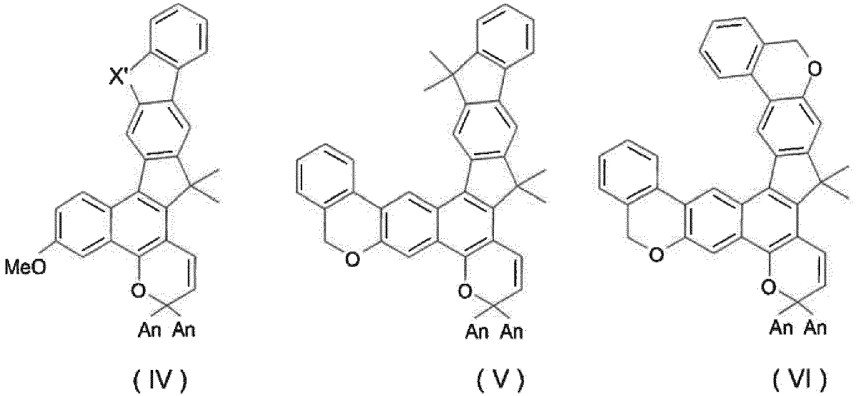 où X' et X sont tels que définis ci-dessus.

10. Utilisation des naphtopyranes photochromiques multi- condensés selon l'une des revendications 1 à 9 dans et sur des matières plastiques.

11. Utilisation selon la revendication 10, dans laquelle la matière plastique est une lentille ophtalmique, en particulier pour des lunettes de conduite.
